# EUROPEAN PATENT SPECIFICATION

(11) **EP 1 492 821 B1**
(45) Date of publication and mention of the grant of the patent: **02.08.2006**
(21) Application number: 03746252.0
(22) Date of filing: 08.04.2003
(51) Int. Cl.: C08B 30/18, A61K 33/26, A61K 31/715, A61P 7/06

(54) **IRON DEXTRIN COMPOUNDS FOR THE TREATMENT OF IRON DEFICIENCY ANAEMIA**
EISEN-DEXTRIN VERBINDUNG ZUR BEHANDLUNG VON ANÄMIEN DURCH EISENMANGEL
COMPOSES DE DEXTRINE DE FER CONCUS POUR TRAITER UNE ANEMIE FERRIPRIVE

(30) Priority: 09.04.2002 DK 200200512
(43) Date of publication of application: 05.01.2005
(73) Proprietor: Pharmacosmos Holding A/S, 4000 Roskilde (DK)
(72) Inventor: ANDREASEN, Hans, Berg, DK-4300 Holbaek (DK); CHRISTENSEN, Lars, DK-4000 Roskilde (DK)
(74) Representative: Rotne, Jens Styrup
(86) International application number: PCT/DK2003/000228
(87) International publication number: WO 2003/087164

(56) References cited:
- GB-A- 1 076 219
- US-A- 4 189 474
- US-B1- 6 291 440

## Description

The invention relates to novel iron dextrin compounds and to processes for the manufacture thereof. Further the invention relates to the use of the iron dextrin for the manufacture of pharmaceutical compositions for the treatment of iron deficiency anaemia in humans or domestic livestock.

### BACKGROUND FOR THE INVENTION

Iron-deficiency anaemia has been described as one of the most common - possibly the most common - pathological conditions among humans when viewed on a global basis. Also in modern farm-breeding of pigs and other domestic animals iron-deficiency anaemia is a problem unless suitable prophylactic measures are taken.

Although iron-deficiency anaemia can often be prevented or cured by oral administration of iron-containing preparations, it is in many cases preferred to use parenterally administrable iron preparations to avoid variations in bio availability of oral administrations and to ensure effective administration.

Therefore, iron-containing preparations for parenteral use, that means subcutaneous, intramuscular or intravenous administration, have for many years been at the disposal of the veterinary or human medical practitioner.

Although various iron-containing substances have been used or suggested as components in parenterally injectable preparations against iron-deficiency anaemia, the most common preparations accepted today are such which comprise a combined product of ferric oxyhydroxide (or ferric hydroxide) in association with dextran. Dextran is a polymeric carbohydrate produced by the microorganisms *Leuconostoc mesenteroides.*

Even though dextran in many ways is a desirable compound it has the disadvantages that it is metabolised only in a limited extend in the human body. Further dextran may give rise to anaphylactic reactions when administered parenterally.

An iron-containing preparation for parenteral injection should obviously satisfy several requirements including ready availability of the iron for haemoglobin synthesis, absence of local or general side-effects and stability on storage enabling a satisfactory shelf-life at ambient temperature.

Often it is desirable to administer an iron preparation orally because this is most convenient for the recipients. A frequent disadvantage encountered after administration of iron preparations orally is impaired digestion. Good iron preparations should provide iron to the body in the gastro intestinal tract in a controlled way in order to provide sufficient iron to be assimilated through the intestinal epithelium and should not have an adverse influence on the digestion as such.

GB 1,076,219 disclose a method for the manufacture of a complex containing iron, low molecular weight dextrin or dextran and sorbitol for the prophylaxis or treatment of iron deficiency anaemia.

US 4,927,756 disclose a procedure for the manufacture of an iron dextran compound wherein the molecular weight of the dextrans is in the range of 2000-4000. It is further stated that dextran and saccharides having a molecular weight below 1000 Da decomposes in the reaction conditions leading to toxic products.

WO 9900160 discloses iron dextrans consisting of dextrans having a weight average molecular weight of 700 to 1400 and number average molecular weight of 400 to 1400 Daltons in stable association with ferric oxyhydroxide. The disclosed iron dextrans complexes give rise to a reduced number of incidences of anaphylactic side effects.

Additional iron preparations for the treatment of iron deficiency anaemia are know such as iron-sucrose and iron-gluconate compounds. These compounds binds iron less tight with the consequence that the concentration of free Fe³⁺ ions are higher which increases the toxicity of the iron compounds when administered parenterally and may lead to disturbance of digestion when administered orally.

It has been described that among the Banthu people the occurrence of iron-deficiency anaemia is very low and contrary incidences of iron overload are frequent. The iron is mainly supplied by the diet as a consequence of the traditional preparation of corn gruel diet in iron pots. Even though no direct evidence have been provided it is assumed that the iron is solubilized by the acid medium of the stomach, mixed with sugars from the hydrolysed dietary starch and then delivered into the small intestine where the bolus, rich in sugar and iron is neutralized to form soluble carbohydrate complexes of the iron. These soluble iron sugar complexes are readily transported and adsorbed by the intestinal tract with no mucosal block observed. (Spiro and Saltman. Polynuclear complexes of Iron and their biological Implications: Structure and bonding pp. 116-156).

Despite the above mentioned progress regarding the manufacture of iron products it is desirable to provide a iron compound useable for the preparation of compositions for the treatment or prophylaxis of iron deficiency anaemia, which iron compound does not provide the problems inherently connected with dextrans.

### DESCRIPTION OF THE INVENTION

It is an object of the present invention to provide new iron preparations for the treatment of iron deficiency anaemia, which preparations fulfil all the following requirements:
- high availability of iron for adsorption in the intestine without causing problems with the digestion, when administered orally;
- providing iron in a form that is readily adsorbed in the intestine;
- high availability of iron without risk for toxicity caused by high local concentration of Fe³⁺, when administered parentarally;
- not connected with anaphylactic reaction;
- comprising a high amount of iron;
- capable of forming stable solutions of said iron preparation comprising a high amount of iron, which solutions fulfil the basal requirements for pharmaceutical compositions, i.e. can be sterilized, preferably by autoclaving, and which are stable during storage for a long period at ambient temperatures.

The present inventors has surprisingly realised that all the above requirements are met by the iron dextrin compounds according to the invention.

According to the present invention an iron(III)-dextrin compound is provided.

The dextrin compound is prepared by hydrolysis of starch. Dextrins are saccharides composed of glucose units linked together predominantly by α-1,4-glucosidic bonds.

Dextrins are usually made by depolymerisation of starch using known depolymerising means such as acids, bases or enzymes. Depending on the origin starch contains also few α-1,6-glucosidic bonds positioned at branch points of a polyglucose chain. Therefore dextrins may also contain a similar low fraction of α-1,6-glucosidic bonds. By adjusting the conditions for the depolymerisation of the starch it may be possible to favour breakage of α-1,4-glucosidic bonds or α-1,6-glucosidic bonds so that the ratio between these types of bonds differs between the originating starch and the prepared dextrins.

One of the characteristic properties of starch and dextrin is their gelling properties. In contrast to dextrans starch and higher dextrins gellify even at modest concentrations, which makes the handling more difficult.

The gelling properties of starch and dextrins can be reduced by reducing the molecular weight by hydrolysis, however, the hydrolysis should not be to extensive as it is known that sugars and small dextrins may give rise to toxicity problems when combined with iron in an association complex.

It is preferred that the starch is hydrolysed until it does not form strong coloured complexes with iodine. Solutions of starch hydrolysed to that extend comprise high amounts of dextrins in the desired molecular size range, and has a viscosity that is sufficient low to allow the handling of the solutions to be easy and accurate.

According to the invention the molecular weight of the starch is preferably performed as an acid hydrolysis, using a strong mineral acid such as sulphuric acid, phosphoric acid or hydrochloric acid. Hydrochloric acid is a preferred acid for the hydrolysis of starch.

Further the inventors have realized that it is desirable to purify the dextrins to a narrow molecular weight distribution in order to obtain more uniform iron-dextrin complexes.

Therefore it is an important feature of the invention that the dextrin is hydrolysed to a suitable low molecular weight, and is fractioned to a narrow range of molecular weights avoiding high molecular weight dextrins and low molecular saccharides.

The weight average molecular weight (Mw) of the dextrins to be combined with iron according to the invention must be less than 3000 Daltons and the number average molecular weight (Mn) must be higher that 400 Daltons.

As the molecular weight of the dextrin must be narrow it is another important feature of the invention that the 10 % fraction of the dextrins having the highest molecular weight has an average molecular weight of less than 4500 Daltons, and that 90 % of the dextrins are having molecular weights of less than 3000 Daltons. It is further important that the 10 % fraction having the lowest molecular weight has a weight average molecular weight of 340 Daltons or more.

In a preferred embodiment the 10% fraction of the dextrins having the highest molecular weights has an average molecular weight less than 4000 Da, 90% of the dextrins having molecular weights of less than 3000 Daltons, and the 10 % fraction having the lowest molecular weights has a weight average molecular weight of 800 Daltons or more.

The present inventors have surprisingly discovered that such a dextrin fraction has a sufficient low viscosity that allow easy and reliable handling of solutions of the dextrin, and further that such a dextrin fraction provides association complexes with iron in a very uniform size.

The fractionation may in principle be done using known procedures for fractionation of oligosaccharides that are suitable for fractioning to a narrow range of molecular weights. Such procedures include chromatographic purification, ion chromatographic methods and purifications using membrane separation technology, where purification by membrane processes is preferred. It is particular preferred that a membrane process using a membrane having a cut-off in the range of 340-800 Daltons is used for removing the low molecular weight saccharides.

In contrast to the fractioning using membrane processes the traditionally used fractionation technique based on precipitations is not a suitable fractionation technique for the present invention, presumably because the dextrin fraction obtained will not be sufficiently narrow. Consequently, iron dextrin compounds prepared using a dextrin fractioned by traditional precipitation will not possess the beneficial properties of the iron dextrin compounds according to the present invention.

Before being combined with iron the reducing capability of the dextrins is removed. This may be done by hydrogenation of the terminal aldehyde groups of the dextrins to alcohols. This reduction may be performed using well known procedures. Hydrogenation using sodium borohydride is preferred.

After the hydrogenation the reducing capability of the dextrins should be less than 3.0 % determined using cupri oxidation method.

The purified and hydrogenated dextrin as an aqueous solution is combined with at least one water soluble ferric salt; base is added to the resulting solution to form ferric hydroxide, and the resulting mixture is heated to transform the ferric hydroxide into ferric oxyhydroxide as an association compound with the dextrin.

A preferred example of a water soluble ferric salt is ferrichloride.

A preferred embodiment of the process comprises the following:
(i) preparing an aqueous solution comprising the purified hydrogenated dextrin and at least one water-soluble ferric salt;
(ii) adjusting the pH of said aqueous solution to a value above 7 by addition of a base;
(iii) heating the mixture to a temperature above 85°C until it turns to a black or dark brown colloidal solution which can be filtered through a 0.45 µm filter; and
(iv) further purification and stabilization using filtration, heating and membrane processes and addition of one or more stabilizers, and optionally drying the solution to obtain the desired iron-dextrin compound as a stable powder.

It is even more preferred that the pH of the aqueous solution in step (ii) is adjusted to a value above 8.5 by addition of a base.

The stabilization suitably takes place by addition of a salt of an organic hydroxy acid, preferably a citrate.

In one preferred aspect the invention relates to an iron dextrin compound being a water soluble powder comprising up to 50% (w/w) iron. Preferably the iron content of the powder is in the range of 10-50% (w/w), more preferred in the range of 20-45% (w/w), and even more preferred in the range of 30-42% (w/w).

The present invention thus deals with iron-dextrin compounds having an extremely low frequency of non-desired side effects and being satisfactory stable, also during sterilization and storage as aqueous solutions, which iron-dextrin compound can be used as component in a pharmaceutical composition for prophylaxis or treatment of iron-deficiency in animal or human subjects by parenteral or oral administration, the iron-dextrin compound being characterized in that it comprises hydrogenated dextrin having a weight average molecular weight (Mw) less than 3,000 Daltons, preferably approximately 1,000 Daltons, a number average molecular weight (Mn) equal to or higher than 400 Daltons in stable association with ferric oxyhydroxide.

Alternatively the drying operation is omitted, and an injection liquid is produced from the purified solution without intermediate drying thereof.

In a further preferred embodiment the hydrogenation of the dextrin is performed by means of sodium borohydride in aqueous solution.

The present inventors have surprisingly discovered that the iron dextrin compound according to the invention possess significant advantages compared with previously known iron dextrin compounds.

First using the method according to the invention it is possible to prepare an iron dextrin having a very high iron content calculated as the ratio between iron and the total iron dextrin complex.

Secondly, the iron dextrin compounds according to the invention are highly soluble in water, which make it possible to prepare aqueous solutions of the iron dextrins according to the invention containing very high amount of iron. These solutions are stable and do not deteriorate by storage such as by gellification or precipitation.

Further solution of the iron dextrin compounds according to the invention can be sterilized by autoclaving without substantial physical changes of the solutions. Thus the solutions may be autoclaved without any significant change of the molecular weights of the complexes or the viscosity of the solution.

Consequently, the iron dextrin compounds according to the invention provides the possibility of preparing pharmaceutical compositions comprising very high amounts of iron per mass unit, which composition fulfils all requirements for pharmaceutical compositions such as being autoclavable and stable for a long period at ambient temperature.

For example injection liquids comprising 20% iron may be prepared according to the invention. Such injection liquids comprising high amount of iron offers the advantage that a smaller amount of the liquid needs to be injected in the subject being treated, which obviously is an advantage for the subject being treated as well for the person performing the treatment.

Thus in a further aspect the invention provides aqueous solutions comprising the iron dextrin compound according to the invention, wherein the iron content is up to 35%. Preferably the iron content is in the range of 1-35%, more preferred in the range of 5-35%, even more preferred in the range of 5-30%, and most preferred in the range of 10-25%. Aqueous solutions comprising 1, 2, 5, 10, 20, 25 or 30% iron are also preferred embodiments of the invention.

The aqueous solutions may be preserved using any recognized preserving techniques such as autoclaving, filtration through a 0.2-0.5 micron filter under sterile conditions or addition a preserving agent. As an example of preserving agents can be mentioned 0.5% phenol.

Autoclaving is a preferred method for preserving the aqueous solutions according to the invention. Particular preferred is autoclaving at a temperature of 121-135°C in a period of 5-40 minutes. If the pH of the aqueous solutions is below 7.5 it is preferred to autoclave the solution for a period of less than 40 minutes.

In a further preferred embodiment said aqueous solutions are pharmaceutical compositions.

The term Pharmaceutical compositions should in the present specification be understood broadly and comprises compositions for treating or preventing iron deficiency anaemia in a human individual or an animal, such as a domestic animal.

Pharmaceutical compositions comprising iron dextrin compounds according to the invention may be prepared using procedures that are well known for skilled person.

In one embodiment injection liquids are prepared by providing an aqueous solution of iron dextrin according to the invention, dilution in a suited solvent if desired, adjustment of pH, sterilizing by filtration and filling into previous sterilized ampoules or vials.

In another embodiment injection liquids are prepared by providing an aqueous solution of iron dextrin according to the invention, dilution in a suited solvent if desired, adjustment of pH, filling into previous sterilized ampoules or vials followed by sterilization by autoclaving of the filled ampoules or vials.

One preferred embodiment of the invention provides an injection liquid intended for the administration in a human comprising 1-20% iron per mass unit of the injection liquid.

Another preferred embodiment of the invention provides an injection liquid intended for the administration in an animal comprising 10-30% iron per mass unit of the injection liquid.

Preparations for oral use may be produced using procedures well known for the person skilled in the art. As examples of preparations for oral use can be mentioned tablets, capsules, syrups, pastes and mixtures.

Pharmaceutical preparations comprising the iron dextrin compound according to the invention may be formulated with additional nutritional or pharmaeutical useful agents, such as vitamins, preferably watersoluble vitamins, micro nutrients such as trace metals e.g. cobalt, copper, zinc or selenium, or antibiotics such as tylosin. Vitamins insoluble in water may even be emulsified into an aqueous solution comprising the iron dextrin compound according to the invention using a suitable emulsifier.

The skilled person will appreciate the advantage that in contrast to dextrans no anaphylactic problems are connected to dextrins. This has the consequence that even if the removal of high molecular weight molecules is less efficient than intended, the risks for induction of adverse reactions in the recipients are minimized because dextrins in all molecular weights are safe and not prone to inducing any anaphylactic reactions.

Surprisingly the iron dextrins according to the invention can bind an equal amount or more iron per weight unit of carbohydrate and be more soluble compared with iron dextrins according to the prior art. Further dextrins contain primary alcohol groups located at the 6-position of the sugar groups, where the protons of the primary alcohol groups may be removed under basic conditions. Without wishing to be bound by any theory it is assumed that the bonding properties of said primary alcohol groups is responsible for the fact that dextrins binds to iron differently that dextrans.

Compared to previous known iron dextrin compounds the iron dextrin compounds according to the invention are more soluble and have less tendency to gellify during manufacture and storage.

When the iron dextrin compound according to the invention is administered orally in a pharmaceutical efficient dose, a satisfactory availability of iron for assimilation in the intestines is provided without any adverse effect on the digestion.

The invention is now further illustrated by the following non-limiting examples.

### EXAMPLES

### Example 1

### Hydrolysis and hydrogenation of dextrin

Gelatine forming dextrin of Mw > 3000 was hydrolysed at pH 1.5 at a temperature of 95°C. The reaction was monitored by taking samples and analysing these chromatographically using gel permeation chromatography.

When the molecular weight of the dextrins had reached a desirable value i.e. weight average molecular weight less than 3000 Da the hydrolysis was terminated, by cooling and neutralisation.

By the hydrolysis low molecular weight dextrins and glucose is formed.

The cool and neutralised dextrin solution was subjected to membrane purification processes having a cut off value of 340 - 800 Da in order to remove glucose and smaller dextrins formed during the hydrolysis, whereafter the content of dextrins was determined using a refractometer and the reducing sugars were determined using cupri oxidation.

The reducing capability (RC) was decreased by treatment with sodium borohydride. After the sodium borohydride treatment the reducing capability was less than 3.0 %.

Next the solution was neutralized to pH < 7.0 and subsequently deionised. The average molecular weight and the molecular weight distribution were determined chromatographically using dextrans as standard.

### Example 2

### Preparation of an iron dextrin compound

A kg of dextrin solution produced as above was mixed with B kg FeCl₃.6H₂O in aqueous solution. To the agitated mixture was added C kg of Na₂CO₃ as a saturated aqueous solution and next the pH was raised to 10.0 using concentrated aqueous NaOH (27 % w/v)(approximately 25 1).

The thus obtained mixture was heated above 85°C until it turned into a black or dark brown colloidal solution, which could be filtered through a 0.45 µm filter and subsequently cooled. After cooling the solution was adjusted to pH 5.8 using concentrated hydrochloric acid (approximately 2-5 1). The solution was purified using membrane processes until the chloride content in the solution was less than 0.15% calculated on basis of a solution containing 5% w/v iron.

If the chloride content of the solution was less than desired sodium chloride was added, the pH value adjusted to 5.6 using hydrochloric acid or sodium hydroxide and the solution was filtered through a 0.45 µm membrane filter.

Finally the solution was spray dried to form an iron-dextrin powder.

For the values of A, B, C see the table below.

### Example 3

### Preparation of an iron-dextrin citrate compound

A kg of dextrin solution prepared as in example 1 was mixed with B kg FeCl₃.6H₂O in aqueous solution. To the agitated mixture was added C kg of Na₂CO₃ as a saturated aqueous solution and next the pH was raised to 10.0 using concentrated aqueous NaOH (27 % w/v)(approximately 25 1).

The thus obtained mixture was heated above 85°C until it turned into a black or dark brown colloidal solution, which could be filtered through a 0.45 µm filter and subsequently cooled. After cooling the solution was adjusted to pH 5.8 using concentrated hydrochloric acid (approximately 2-5 1). The solution was purified using membrane processes until the chloride content in the solution was less than 0.15% calculated on basis of a solution containing 5% w/v iron.

Citric acid in an amount of D kg was added and the pH was adjusted to above 8.0 using sodium hydroxide and the solution was stabilized by raising the temperature to above 100°C for 60 minutes.

Subsequently the pH value was adjusted using hydrochloric acid to pH 5.6. In case that the chloride content of the solution was less than desired it was adjusted using sodium chloride.

Thereafter the solution was filtered through a 0.45 µm membrane filter and spray dried to form a iron dextrin powder.

**Table I. Iron dextrin compounds prepared according to example 1-3**

| Batch | A | Mw | Mn | RC | B | C | D |
|---|---|---|---|---|---|---|---|
| | kg | Da | Da | % | kg | kg | kg |
| TS 423 | 207 | 1500 | 860 | 2.8 | 150 | 80 | 0 |
| TS 424 | 207 | 1500 | 860 | 2.8 | 150 | 80 | 3.6 |
| TS 425 | 138 | 1500 | 860 | 2.8 | 150 | 80 | 3.6 |
| TS 426 | 120 | 2150 | 910 | 2.6 | 150 | 80 | 3.6 |
| TS 427 | 120 | 2150 | 910 | 2.6 | 150 | 80 | 0 |
| TS 501 | 105 | 1544 | 840 | 2.6 | 150 | 80 | 3.6 |

### Example 4

### Analysis of prepared iron dextrin compounds

The iron dextrin preparations were analysed for the chemical composition. The results appear in table II below.

Further the molecular weight of the formed complexes was measured using gel permeation chromatography. No free iron was detected in solutions comprising the complexes.

**Table II. Analysis data for the complexes**

| Batch | iron content % | dextrin content % | Mp |
|---|---|---|---|
| TS 423 | 24.7 | 56.8 | 465,000 |
| TS 424 | 23.1 | 50.2 | 313,000 |
| TS 425 | 28.6 | 40.0 | 270,000 |
| TS 426 | 36.9 | 37.6 | 282,000 |
| TS 427 | 33.3 | 37.6 | 240,000 |
| TS 501 | 35.1 | N.A. | 284,000 |

| | | | |
|---|---|---|---|
| N.A. = not analysed | | | |

### Example 5

### Toxicity test of iron dextrin preparations

The preparations disclosed in table I prepared as a aqueous solution containing 2 % Fe(III) could be autoclaved at 120°C without adverse effects.

Further the final preparations when prepared as aqueous solutions could pass the test for abnormal toxicity performed in accordance with USP 24.

### Example 6

The iron dextrins listed in Table III were prepared using the following general procedure.

### General Procedure

A kg of a dextrin solution (Batch T02013-1) having a reducing capability (RC) of 1.05%, prepared as in example 1, was mixed with B kg FeCl₃,6H₂O in aqueous solution. To the agitated mixture was added Cₐ kg of Na₂CO₃ as a saturated aqueous solution and next the pH was raised to 10 by adding of C_{b} 1 of concentrated NaOH (27% w/v).

The thus obtained mixture was heated above 85°C until it turned into a black or dark brown colloidal solution, which could be filtered through a 0.45 µm filter. After cooling the solution was adjusted to pH approximately 6.0 (5.0 - 7.0) using concentrated hydrochloric acid. The solution was purified using membrane separation until the chloride content in the solution was less than 0.15% calculated on the basis of a solution containing 5% w/v iron.

Citric acid in an amount of D kg was added and the pH was adjusted to above 8.0 using sodium hydroxide and the solution was stabilized by raising the temperature to above 100°C for 60 minutes.

Subsequently the pH was adjusted using hydrochloric acid to approximately 7.5 (6.0-9.0). In case that the chloride content of the solution was less than desired it was adjusted using sodium chloride.

Next the solution was filtered through a 0.45 µm filter and spray dried to form an iron dextrin powder.

**Table III**

| Iron dextrins prepared in example 6 | | | | | | | | |
|---|---|---|---|---|---|---|---|---|
| Batch | A kg | M_{w} Da | Mₙ Da | RC % | B kg | Cₐ kg | C_{b} l | D kg |
| TZ 122 | 105 | 1250 | 860 | 1.05 | 300 | 173 | 45 | 7.2 |
| TZ 121 | 105 | 1250 | 860 | 1.05 | 250 | 144 | 45 | 6.0 |
| TZ 118 | 105 | 1250 | 860 | 1.05 | 200 | 116 | 25 | 4.8 |
| TZ 120 | 105 | 1250 | 860 | 1.05 | 150 | 87 | 20 | 3.6 |

Similar iron dextrins were prepared using same amounts of ingredients but without citric acid (data not shown)

### Example 7

The iron dextrin preparations from example 6 were analysed for the chemical composition. The results appear in Table IV below.

Further the molecular weight of the formed complexes was measured using gel permeation chromatography. No free iron was detected in the solution comprising the complexes.

**Table IV**

| Analytical data for iron dextrin powder | | |
|---|---|---|
| Batch | Iron content % | dextrin content % |
| TZ 122 | 40.0 | 23.3 |
| TZ 121 | 38.6 | 27.2 |
| TZ 118 | 35.8 | 29.7 |
| TZ 120 | 31.6 | 38.6 |

### Example 8

The iron dextrin compounds prepared in example 6 were used for the preparation of aqueous solutions containing 100 mg iron(III)/ml (10 %) and 200 mg iron(III)/ml (20%). The solutions were analysed before and after 20 and 40 minutes of autoclaving at 121°C. The iron dextrin solutions appear to be unchanged following autoclaving. Results are shown in Table V to VIII below.

**Table V**

| Analytical data for 10% w/v iron dextrin solution Relative viscosity measured prior to, after 20 minutes and 40 minutes autoclaving at 121°C. | | | |
|---|---|---|---|
| Batch | before autoclaving | 20 minutes autoclaving | 40 minutes autoclaving |
| TZ 122 | 2.42 | 2.32 | 2.63 |
| TZ 121 | 2.61 | 2.38 | 2.65 |
| TZ 118 | 2.77 | 2.82 | 2.70 |
| TZ 120 | 3.51 | 3.21 | 3.36 |

**Table VI**

| Analytical data for 10 % iron dextrin solution. Measured peak molecular weight before autoclaving after 20 minutes and after 40 minutes autoclaving. | | | |
|---|---|---|---|
| Batch | before autoclaving | 20 minutes autoclaving | 40 minutes autoclaving |
| TZ 122 | 229,000 | 224,000 | 234,000 |
| TZ 121 | 206,000 | 207,000 | 215,000 |
| TZ 118 | 206,000 | 209,000 | 208,000 |
| TZ 120 | 184,000 | 188,000 | 191,000 |

**Table VII**

| Analytical data for 20% iron dextrin solution Relative viscosity measured prior to, after 20 minutes and 40 minutes autoclaving at 121°C. | | | |
|---|---|---|---|
| Batch | before autoclaving | 20 minuttes autoclaving | 40 minuttes autoclaving |
| TZ 122 | 9.62 | 9.37 | 10.98 |
| TZ 121 | 10.9 | 10.7 | 11.0 |
| TZ 118 | 12.61 | 13.2 | 13.2 |
| TZ 120 | 23.3 | 22.8 | 22.4 |

**Table VIII**

| Analytical data for 20 % iron dextrin solution. Measured peak molecular weight before autoclaving, after 20 minutes and after 40 minutes autoclaving. | | | |
|---|---|---|---|
| Batch | before autoclaving | 20 minutes autoclaving | 40 minutes autoclaving |
| TZ 122 | 227,000 | 229,000 | 219,000 |
| TZ 121 | 208,000 | 213,000 | 239,000 |
| TZ 118 | 206,000 | 213,000 | 217,000 |
| TZ 120 | 185,000 | 194,000 | 194,000 |

Further the preparations could pass the test for abnormal toxicity performed in accordance with USP 24.

## Claims

1. Iron dextrin compound consisting of hydrogenated dextrin in stable association with ferric oxohydroxide, **characterized in that**, said hydrogenated dextrin has a weight average molecular weight (Mw) equal to or less than 3,000 Daltons and a number average molecular weight higher than or equal to 400 Daltons, wherein the 10% fraction of said hydrogenated dextrin having the highest molecular weight has a weight average molecular weight of less than 4,500 Daltons, and that 90 % of the dextrins are having a molecular weight of less than 3,500 Daltons, and wherein the 10% fraction of said hydrogenated dextrin having the lowest molecular weight has a weight average molecular weight of 340 Daltons or more.

2. Iron dextrin compound according to claim 1, wherein said dextrin is having a Mw of approximately 1,000 Daltons.

3. Iron dextrin compound according to claim 1, being a powder having an iron content in the range of 10-45% (w/w).

4. Aqueous solution of an iron dextrin compound according to claim 1, wherein the iron content is in the range of 1-30% (weight/vol).

5. Aqueous solution of an iron dextrin compound according to claim 4, wherein the iron content is in the range of 5-25% (weight/vol).

6. Process for preparing the iron dextrin compound of claim 1, comprising the steps of:
(a) hydrolysing starch or dextrin so as to reduce its molecular weight until the hydrolysed starch or dextrin does not form strong coloured complexes with iodine,
(b) hydrogenating the resulting hydrolysed dextrin to convert functional aldehyde groups into alcohol groups,
(c) fractioning of the hydrogenated hydrolysed mixture according to size so that the purified fraction is having a weight average molecular weight equal to or less than 3,000 Daltons, and a number average molecular weight of equal to or higher than 400 Daltons, wherein the 10% fraction of said hydrogenated dextrin having the highest molecular weight has a weight average molecular weight of less than 4,500 Daltons, and that 90 % of the dextrins are having a molecular weight of less than 3,500 Daltons, and wherein the 10% fraction of said hydrogenated dextrin having the lowest molecular weight has a weight average molecular weight of 340 Daltons or more.
(d) combining the resultant fractionated hydrogenated dextrin as an aqueous solution with at least one water soluble ferric salt,
(e) adding base to the resulting aqueous solution to adjust the pH value of the solution to a value higher than 7.0, in order to form ferric hydroxide, and
(f) heating the resultant basic solution to transform the ferric hydroxide into ferric oxyhydroxide in association with said dextrin.

7. The process according to claim 6, wherein the fractioning in step (c) is performed using membrane processes.

8. The process according to claim 6 or 7, wherein in step (e) the resulting solution is adjusted to a pH above 8.5 using said base, and wherein in step (f) heating is carried out at a temperature above 85°C until the solution turns into a black or dark brown colloidal solution, which is then filtered through a 0.45 µm membrane; and thereafter a stabilizer is added, and optionally the solution is dried to obtain a stable powder.

9. The process according to claim 8, wherein the stabilizer is a salt of an organic hydroxy acid.

10. The process according to claim 9, wherein the stabilizer is a salt of a citrate.

11. The process according to any of claims 6 to 7, wherein said hydrogenation in step (b) is performed using sodium borohydride in aqueous solution.

12. The process according to any of claims 6 to 11, wherein the at least one water soluble ferric salt is ferric chloride.

13. A pharmaceutical composition for the treatment or prophylaxis of iron deficiency anaemia in an animal or a human subject, comprising a pharmaceutical efficient amount of the iron dextrin compound of claim 1, wherein the pharmaceutical composition is prepared for parenteral or oral administration.

14. The pharmaceutical composition according to claim 13, where the composition is formulated as tablets, capsules, paste, granulate, solution, mixture or injection liquid.

15. The pharmaceutical composition according to claim 13, wherein the composition is an aqueous solution for parenteral administration having an iron content up to 20% w/v.

16. The pharmaceutical composition according to claim 15, wherein the composition is intended for administration in a human being and comprising 1-20 % iron.

17. The pharmaceutical composition according to claim 16, wherein the composition comprises 2-10% iron.

18. The pharmaceutical composition according to claim 17, wherein the composition comprises 2, 5 or 10% iron.

19. The pharmaceutical composition according to claim 13, wherein the composition is intended for administration in an animal and comprises 1-30 % iron.

20. The pharmaceutical composition according to claim 19, wherein the composition comprises 10-20% iron.

21. The pharmaceutical composition according to any of claims 13-20, further comprising one or more nutritional or pharmaeutical useful agents

22. The pharmaceutical composition according to claim 21, wherein the nutritional or pharmaeutical useful agents are selected from vitamins, copper, cobalt, zinc, or selenium.

23. The pharmaceutical composition according to claims 21, wherein water insoluble vitamins are emulsified using an emulsifier.

24. Process for preparing a pharmaceutical composition of claim 13, comprising dissolving or dispersing the iron dextrin compound in an aqueous liquid.

25. Process according to claim 24, wherein the resulting solution or dispersion is sterilized by filtration, and thereafter filled into previously sterilized ampoules or vials.

26. Process according to claim 24, wherein the resulting solution or dispersion is filled into ampoules or vials followed by autoclaving the filled ampoules or vials.

## Patentansprüche

1. Eisen-Dextrin-Koordinationsverbindung bestehend aus hydrogeniertem Dextrin in fester Koordinationsverbindung mit Eisenoxohydroxid, **dadurch gekennzeichnet, dass** das Dextrin ein Gewichtsmittelmolekulargewicht (MW) von gleich oder unter 3.000 Dalton und ein Zahlenmittelmolekulargewicht von über oder gleich 400 Dalton aufweist, wobei die 10%-Fraktion des hydrogenierten Dextrins mit dem höchsten Molekulargewicht ein Gewichtsmittelmolekulargewicht von unter 4.500 Dalton aufweisen, und 90% der Dextrine ein Molekulargewicht von unter 3.500 Dalton aufweisen, und wobei die 10%-Fraktion des hydrogenierten Dextrins mit dem niedrigsten Molekulargewicht ein Gewichtsmittelmolekulargewicht von 340 Dalton oder darüber aufweisen.

2. Eisen-Dextrin-Koordinationsverbindung nach Anspruch 1, wobei das Dextrin ein MW von annäherungsweise 1.000 Dalton aufweist.

3. Eisen-Dextrin-Koordinationsverbindung nach Anspruch 1, die ein Pulver mit einem Eisengehalt im Bereich von 10-45% (w/v) ist.

4. Wässrige Lösung aus einer Eisen-Dextrin-Koordinationsverbindung nach Anspruch 1, wobei das Eisengehalt im Bereich von 1-30% (w/v) liegt.

5. Wässrige Lösung aus einer Eisen-Dextrin-Koordinationsverbindung nach Anspruch 4, in welcher das Eisengehalt im Bereich von 5-25% (w/v) liegt.

6. Verfahren zur Herstellung der im Anspruch 1 angegebenen Eisen-Dextrin-Koordinationsverbindung, umfassend folgende Schritte:
(a) Hydrolysierung des Amylums oder Dextrins, um sein Molekulargewicht zu reduzieren, bis das hydrolysierte Amylum oder Dextrin keine stark gefärbten Komplexe mit Jod bildet,
(b) Hydrogenierung des sich ergebenden hydrolisierten Dextrins zur Umwandlung funktioneller Aldehydgruppen in Alkoholgruppen,
(c) Fraktionierung des hydrogenierten hydrolysierten Gemisches nach Größe, so dass die gereinigte Fraktion ein Gewichtsmittelmolekulargewicht von gleich oder unter 3.000 Dalton und ein Zahlenmittelmolekulargewicht von gleich oder über 400 Dalton aufweist, wobei die 10%-Fraktion des hydrogenierten Dextrins mit dem höchsten Molekulargewicht ein Gewichtsmittelmolekulargewicht von unter 4.500 Dalton und 90% der Dextrine ein Molekulargewicht von unter 3.500 Dalton aufweisen, und wobei die 10%-Fraktion des hydrogenierten Dextrins mit dem niedrigsten Molekulargewicht ein durchschnittliches Gewichtsmittelmolekulargewicht von 340 Dalton oder darüber aufweisen.
(d) Kombinierung des sich ergebenden fraktionierten, hydrogenierten Dextrins als wässrige Lösung mit mindestens einem wasserlöslichen Eisensalz,
(e) Zusatz einer Base zur sich ergebenden wässrigen Lösung zur Einstellung des pH-Wertes der Lösung auf einen Wert höher als 7,0 zur Bildung von Eisenhydroxid, und
(f) Erhitzen der sich ergebenden basischen Lösung zur Umwandlung des Eisenhydroxids in Eisenoxyhydroxid in Koordinationsverbindung mit dem genannten Dextrin.

7. Verfahren nach Anspruch 6, wobei die Fraktionierung im Schritt (c) unter Verwendung von Membranverfahren durchgeführt wird.

8. Verfahren nach den Ansprüchen 6 oder 7, wobei im Schritt (e) die sich ergebende Lösung unter Verwendung der genannten Base auf einen pH-Wert von über 8,5 eingestellt wird, und wobei im Schritt (f) das Erhitzen bei einer Temperatur von über 85°C durchgeführt wird, bis sich die Lösung in eine schwarze oder dunkelbraune kolloidale Lösung verwandelt, die dann durch eine 0.45 µm Membran filtriert wird; und anschließend ein Stabilisator zugesetzt wird, und die Lösung wahlweise zum Erhalten eines festen Pulvers getrocknet wird.

9. Verfahren nach Anspruch 8, wobei der Stabilisator ein Salz einer organischen Hydroxysäure ist.

10. Verfahren nach Anspruch 9, wobei der Stabilisator ein Salz eines Zitrats ist.

11. Verfahren nach einem der Ansprüche 6 bis 7, wobei die genannte Hydrogenierung im Schritt (b) unter Verwendung von Natriumborhydrid in wässriger Lösung durchgeführt wird.

12. Verfahren nach einem der Ansprüche 6 bis 11, wobei mindestens ein wasserlösliches Eisensalz Eisenchlorid ist.

13. Pharmazeutisches Präparat zur Behandlung oder Prophylaxe von Anämien durch Eisenmangel in einem tierischen oder menschlichen Subjekt, umfassend eine pharmazeutisch wirksame Menge der im Anspruch 1 angegebenen Eisen-Dextrin-Koordinationsverbindung, wobei das pharmazeutische Präparat zur parenteralen oder oralen Verabreichung hergestellt wird.

14. Pharmazeutisches Präparat nach Anspruch 13, wobei das Präparat als Tabletten, Kapseln, Paste, Granulat, Auflösung, Gemisch oder injektionsflüssigkeit formuliert ist.

15. Pharmazeutisches Präparat nach Anspruch 13, wobei das Präparat eine wässrige Lösung zur parenteralen Verabreichung mit einem Eisengehalt von bis zu 20% w/v ist.

16. Pharmazeutisches Präparat nach Anspruch 15, wobei das Präparat zur Verabreichung in einen Mensch vorgesehen ist und 1-20% Eisen enthält.

17. Pharmazeutisches Präparat nach Anspruch 16, wobei das Präparat 2-10% Eisen enthält.

18. Pharmazeutisches Präparat nach Anspruch 17, wobei das Präparat 2, 5 oder 10% Eisen enthält.

19. Pharmazeutisches Präparat nach Anspruch 13, wobei das Präparat zur Verabreichung in ein Tier vorgesehen ist und 1-30% Eisen enthält.

20. Pharmazeutisches Präparat nach Anspruch 19, wobei das Präparat 10-20% Eisen enthält.

21. Pharmazeutisches Präparat nach einem der Ansprüche 13 bis 20, das außerdem einen oder mehrere ernährungsmäßig oder pharmazeutisch verwendbaren Wirkstoffe enthält.

22. Pharmazeutisches Präparat nach Anspruch 21, wobei die ernährungsmäßig oder pharmazeutisch verwendbaren Wirkstoffe aus Vitaminen, Kupfer, Kobalt, Zink oder Selen ausgewählt sind.

23. Pharmazeutisches Präparat nach Anspruch 21, wobei wasserunlösliche Vitamine unter Verwendung eines Emulgators emulgiert werden.

24. Verfahren zur Herstellung eines im Anspruch 13 angegebenen pharmazeutischen Präparats, umfassend die Auflösung oder Dispergierung der Eisen-Dextrin-Koordinationsverbindung in einer wässrigen Flüssigkeit.

25. Verfahren nach Anspruch 24, wobei die sich ergebende Lösung oder Dispersion durch Filtrierung steralisiert und anschließend in vorsterilisierte Ampullen oder Phiolen gefüllt wird.

26. Verfahren nach Anspruch 24, wobei die sich ergebende Lösung oder Dispersion in Ampullen oder Fläschchen gefüllt wird, gefolgt von einer Autoklavierung der gefüllten Ampullen oder Phiolen.

## Revendications

1. Composé de dextrine dé fer constitué de dextrine hydrogénée en combinaison stable avec oxyhydroxyde ferrique, **caractérisé en ce que** la dite dextrine hydrogénée présente un poids moléculaire (Pm) moyen en poids égal ou inférieur à 3.000 daltons et un poids moléculaire moyen en nombre supérieur ou égal à 400 daltons, dans lequel le fractionnement de 10 pour cent de la dite dextrine hydrogénée ayant le plus haut poids moléculaire présente un poids moléculaire moyen en poids inférieur à 4.500 daltons, et **en ce que** 90 pour cent des dextrines présentent un poids moléculaire inférieur à 3.500 daltons, et dans lequel le fractionnement de 10 pour cent de la dite dextrine hydrogénée présente un poids moléculaire moyen en poids de 340 daltons ou plus.

2. Composé de dextrine de fer selon la revendication 1, où la dite dextrine présente un Pm d'environ 1.000 daltons.

3. Composé de dextrine de fer selon la revendication 1, étant une poudre présentant une teneur en fer avoisinant 10 à 45 pour cent (p/v).

4. Solution aqueuse d'un composé de dextrine de fer selon la revendication 1, dans laquelle la teneur en fer est de l'ordre de 1 à 30 pour cent (poids/volume).

5. Solution aqueuse d'un composé de dextrine de fer selon la revendication 4, dans laquelle la teneur en fer est de l'ordre de 5 à 25 pour cent (poids/volume).

6. Procédé pour la préparation d'un composé de dextrine de fer selon la revendication 1, comprenant les étapes:
(a) d'hydrolyser de l'amidon ou de la dextrine à manière de réduire son poids moléculaire jusqu'à ce que l'amidon ou la dextrine hydrolysé ne forme pas de complexes fortement colorés avec iode,
(b) d'hydrogéner la dextrine hydrolysée résultante pour convertir des groupements fonctionnels aldéhydes en groupements alcools,
(c) de fractionner la combinaison hydrolysée et hydrogénée par ordre d'importance si bien que le fractionnement purifié présente un poids moléculaire moyen en poids égal ou inférieur à 3.000 daltons, et un poids moléculaire moyen en nombre égal ou supérieur à 400 daltons, dans lequel le fractionnement de 10 pour cent de la dite dextrine hydrogénée ayant le plus haut poids moléculaire présente un poids moléculaire moyen en poids inférieur à 4.500 daltons, et que 90 pour cent des dextrines présentent un poids moléculaire inférieur à 3.500 daltons, et dans lequel le fractionnement de 10 pour cent de la dextrine hydrogénée ayant le plus bas poids moléculaire présente un poids moléculaire moyen en poids de 340 daltons ou plus,
(d) de combiner la dextrine hydrogénée fractionnée résultante en tant qu'une solution aqueuse avec au moins un sel ferrique hydrosoluble,
(e) d'additionner de la base à la solution aqueuse résultante pour régler la valeur pH de la solution à une valeur supérieure à 7,0 afin de former de l'hydroxyde ferrique, et
(f) de réchauffer la solution de base résultante pour transformer l'hydroxyde ferrique en oxyhydroxyde en combinaison avec la dite dextrine.

7. Procédé selon la revendication 6, dans lequel le fractionnement dans l'étape (c) est réalisé par à l'usage de procédés de membrane.

8. Procédé selon la revendication 6 et 7, dans lequel dans l'étape (e), la solution résultante est réglée à un pH supérieur à 8,5 à l'aide de la dite base, et dans lequel dans l'étape (f), le réchauffement est effectué à une température supérieure à 85 °C jusqu'à ce que la solution devienne une solution colloïdale noire ou marron foncé qui est ensuite filtrée à travers une membrane de 0,45 µm, et puis un agent stabilisateur est additionné, et à titre facultatif, la solution est séchée pour obtenir une poudre stable.

9. Procédé selon la revendication 8, dans lequel l'agent stabilisateur est un sel d'un acide hydroxyde organique.

10. Procédé selon la revendication 9, dans lequel l'agent stabilisateur est un sel d'un citrate.

11. Procédé selon l'une quelconque des revendications 6 à 7, dans lequel la dite hydrogénation dans l'étape (b) est effectuée à l'usage d'hydrure borique de sodium en solution aqueuse.

12. Procédé selon l'une quelconque des revendications 6 à 11, dans lequel l'au moins un sel ferrique hydrosoluble est un chlorure ferrique.

13. Composition pharmaceutique pour le traitement ou la prophylaxie de l'anémie ferriprive dans un sujet humain ou animal, comprenant une quantité efficace pharmaceutique du composé de dextrine de fer selon revendication 1, dans laquelle la composition pharmaceutique est préparée à l'administration parentérale ou orale.

14. Composition pharmaceutique selon la revendication 13, dans laquelle la composition est formulée comme des comprimés, capsules, pâtes, granulées, solutions, mélanges ou liquides d'injection.

15. Composition pharmaceutique selon la revendication 13, dans laquelle la composition est une solution aqueuse pour administration parentérale ayant une teneur en fer jusqu'à 20 pour cent p/v.

16. Composition pharmaceutique selon la revendication 15, dans laquelle la composition est conçue pour administration dans un être humain et comprend 1-20 pour cent de fer.

17. Composition pharmaceutique selon la revendication 16, dans laquelle la composition comprend 2-10 pour cent de fer.

18. Composition pharmaceutique selon la revendication 17, dans laquelle la composition comprend 2, 5 ou 10 pour cent de fer.

19. Composition pharmaceutique selon la revendication 13, dans laquelle la composition est conçue pour administration dans un être animal et comprend 1-30 pour cent de fer.

20. Composition pharmaceutique selon la revendication 19, dans laquelle la composition comprend 10-20 pour cent de fer.

21. Composition pharmaceutique selon l'une quelconque des revendications 13-20, en plus comprenant un ou plusieurs agents nutritifs ou pharmaceutiques utiles.

22. Composition pharmaceutique selon la revendication 21, dans laquelle les agents nutritifs ou pharmaceutiques utiles sont choisis parmi les vitamines, le cuivre, le cobalt, le zinc ou le sélénium.

23. Composition pharmaceutique selon la revendication 21, dans laquelle des vitamines hydrosolubles sont émulsionnées à l'aide d'un émulsifiant.

24. Procédé pour la préparation d'une composition pharmaceutique selon la revendication 13, comprenant la dissolution ou le dispersement du composé de dextrine de fer dans un liquide aqueuse.

25. Procédé selon la revendication 24, dans lequel la solution ou dispersion résultante est stérilisée par filtrage, et ensuite introduite dans des ampoules ou fioles déjà stérilisées.

26. Procédé selon la revendication 24, dans lequel la solution ou dispersion résultante est introduite dans des ampoules ou fioles, ce qui est suivi par l'autoclave des ampoules ou fioles remplies.
